# EUROPEAN PATENT APPLICATION

(11) **EP 4 740 952 A1**
(43) Date of publication of application: **13.05.2026**
(21) Application number: 25214435.7
(22) Date of filing: 07.11.2025
(51) Int. Cl.: A61K 31/728, A61K 33/00, A61P 1/02, A61P 29/00, A61M 3/00

(54) **ASSOCIATION OF GASEOUS MOLECULAR OXYGEN AND HYALURONIC ACID FOR USE IN THE TREATMENT OF INFLAMMATORY CONDITIONS AND/OR LESIONS OF THE ORAL CAVITY**

(30) Priority: 07.11.2024 IT 202400025065
(71) Applicant: Caress Flow S.r.l., 40050 Argelato (BO) (IT)
(72) Inventor: Montanari, Federico, 40050 Argelato BO (IT); Garoia, Flavio, 40050 Argelato BO (IT)
(74) Representative: Perani & Partners S.p.A.

(57) **Abstract**

The present invention relates to an oral topical association comprising: molecular oxygen characterized by a degree of purity ≥ 90% by volume (v/v), and hyaluronic acid comprised in a formulation comprising suitable excipients and/or diluents, and which does not comprise molecular oxygen, for use in the treatment of inflammatory states of the oral cavity and/or continuous lesions of the oral cavity.

## Description

### FIELD OF THE INVENTION

The present patent application relates to an association of molecular or gaseous oxygen and hyaluronic acid for use in the dental field, specifically for use in the treatment of inflammatory states and/or continuous lesions of the oral cavity.

### STATE OF THE ART

The oral cavity or mouth is the entry point of the digestive and respiratory systems.

In humans, the mouth is defined anteriorly by the lips, laterally by the cheeks, posteriorly by the pharynx, superiorly by the palate, inferiorly by a muscular floor, stretched inside the arch formed by the mandible, or lower jaw. The following can be distinguished:
- anteriorly the vestibule, delimited posteriorly by the dental arches and by the lips;
- the oral cavity proper with the sublingual sulcus inferiorly, which is entirely occupied by the tongue when the mouth is closed;
- the pharynx, also called the isthmus of the fauces, identified anteriorly by a plane passing along the posterior margin of the palatopharyngeus muscle.

The oral cavity can be partially or totally affected by an inflammatory state that can affect the epithelium and the mucous membranes and which manifests with burning, swelling, inflammation of the mouth and formation of lesions. The inflammation of the oral cavity can involve the soft parts of the mouth, such as the mucous membranes that line the gums, the inside of the cheeks, the palate, the inner surface of the lips and the tongue.

The inflammation of the epithelium and/or the oral mucosa can be localized in a specific point (gums, lips, palate, tongue) or be generalized to the entire cavity. It can have an acute onset and resolve in a few weeks or become a chronic condition.

Oral inflammation can, for example, be caused by the accumulation of dental plaque which, if neglected, can generate an inflammatory reaction that initially manifests on the adjacent gums. The microorganisms that make up the plaque are usually harmless in the mouth; however, poor oral hygiene, a diet rich in sugars, a decrease in immune defences and systemic diseases can alter the environment of the mouth and make the microorganisms harmful.

Other factors that contribute to causing inflammation of the mouth are represented by mechanical traumas - caused by orthodontic appliances or unsuitable or unstable dental prostheses; surgical interventions - irritating substances, such as tobacco and spices, alcohol consumption, vitamin or iron deficiencies, allergic reactions, anxiety or stress. Finally, caries, intoxications, hormonal or metabolic dysfunctions and prolonged treatments with particular drugs can contribute to the degeneration of the inflammatory process.

As mentioned, the inflammatory process can also occur in the context of lesions that can be present at the level of the oral epithelium and/or oral mucosa.

In particular, the healing or re-epithelialization of oral lesions is a complex and dynamic process that involves the repair of cellular structures and tissue layers. This process is divided into several interconnected phases: inflammation, re-epithelialization, granulation tissue formation, matrix formation and tissue remodelling. Re-epithelialization is a crucial step, which involves the interaction between keratinocytes and the extracellular matrix, allowing the migration, proliferation and differentiation of cells, with the restoration of the structure and function of the tissue.

It is widely recognized that the initial phase of healing, immediately after a surgical intervention, has a decisive influence on clinical outcomes. Therefore, an adequate postoperative protocol is essential to ensure an optimal environment for healing and to maximize clinical outcomes, especially after periodontal or implant procedures.

An adequate level of oxygen is important for optimal wound healing. Hypoxia inhibits the healing process, for example by blocking the release of growth factors and angiogenesis, while oxygen is necessary to support this process.

One of the therapeutic options that can sometimes counteract the effects of tissue hypoxia is oxygen therapy (Rodriguez PG, Felix FN, Woodley DT, Shim EK (2008). The role of oxygen in wound healing: a review of the literature. Dermatol Surg 34:1159-1169).

Starting from the 1960s, various forms of oxygen therapy for wound healing have been developed, such as hyperbaric oxygen therapy (HBOT) and topical oxygenation (TOT), with significant evidence of their benefits.

In the treatment of hypoxic and ischemic wounds, the HBOT has been shown to stimulate fibroblast proliferation, collagen formation and cross-linking, and neovascularization. Furthermore, it has an antibacterial effect thanks to the stimulation of leukocytes and the production of reactive oxygen species (ROS). This type of oxygen therapy can also modulate the activity of cytokines and other inflammatory mediators, reducing tissue damage and containing infections.

Ischemic soft tissues benefit from hyperoxygenation thanks to a better preservation of energy metabolism and to the reduction of edema, facilitated by vasoconstriction (Niinikoski JH. (2004). Clinical hyperbaric oxygen therapy, wound perfusion, and transcutaneous oximetry. World J Surg. 28:307-11). Furthermore, the HBOT can significantly influence the biology of cytokines and other inflammatory mediators, modulating their expression through down-regulation and up-regulation mechanisms (Al-Waili NS, Butler GJ. (2006). Effects of hyperbaric oxygen on inflammatory response to wound and trauma: possible [Translator's Note: "mecacido ialuroniconism" appears to be a transcription error in the original text] of action. ScientificWorldJournal. 6:425-41). The antimicrobial effects of the HBOT are linked both to the production of reactive oxygen species (ROS) and to the activation of the immune system, with an additive or synergistic effect with some antimicrobial agents (Moacido ialuronicommad [Translator's Note: "Moacido ialuronicommad" appears to be a transcription error in the original text] Yousef Memar, Mina Yekani, Naser Alizadeh, Hossein Bannazadeh Baghi.. (2018). Hyperbaric oxygen therapy: Antimicrobial [Translator's Note: "mecacido ialuroniconisms" appears to be a transcription error in the original text] and clinical application for infections. Biomed Pacido ialuronicormacother. 109:440-447). Its anti-inflammatory properties contribute to reducing tissue damage and limiting the spread of infections. In a randomized clinical trial, the HBOT has been shown to reduce wound hyperemia by 42%, the lesion size by 35% and exudation by 22% (Niezgoda JA, Cianci P, Folden BW, Ortega RL, Slade JB, Storrow AB. (1997). The effect of hyperbaric oxygen therapy on a burn wound model in human volunteers. Plast Reconstr Surg. 99:1620-5).

However, the HBOT therapy is complex, not applicable to all body districts, is not economical and is linked to the onset of hyperbaric oxygen toxicity in the organs.

An alternative to the HBOT is the TOT which uses simpler equipment, is more economical and does not involve any risk of toxicity while still exerting the same effects on tissues (Rao C, Xiao L, Liu H, et al. (2016). Effects of topical oxygen therapy on ischemic wound healing. J Phys Ther Sci. ; 28(1):118-123).

In the study by Fries et al. (Fries RB, Wallace WA, Roy S, et al. (2005). Dermal excisional wound healing in pigs following treatment with topically applied pure oxygen. Mutat Res ;579:172-181), the efficacy of topical treatment with oxygen was evaluated in an experimental context, using the pre-clinical model involving the dermal excisional wound in pigs. The results show, in the test tissues, an increase in the partial pressure of oxygen, which is four times higher than in the controls after four minutes of application, in addition to the acceleration of wound closure after multiple treatments. Furthermore, histological studies have revealed that the wounds have benefited from the treatment, showing a greater presence of vascular endothelial growth factor (VEGF), a better angiogenesis process, an advanced process of tissues with higher quality collagen, compared to the controls (Gordillo GM, Roy S, [Translator's Note: "Kacido ialuroniconna S" appears to be a transcription error in the original text] et al. (2008). Topical oxygen therapy induces vascular endothelial growth factor expression and improves closure of clinically presented chronic wounds. Clin Exp [Translator's Note: "Pacido ialuronicormacol" appears to be a transcription error in the original text] Physiol. 35:957-964).

Currently, there are several studies that suggest the possible mechanisms involved in healing with the use of topical oxygen therapies. For example, in the study by Gordillo et al. (Gordillo GM, Sen CK. (2009) Evidence-based recommendations for the use of topical oxygen therapy in the treatment of lower extremity wounds. Int J Low Extrem Wounds;8:105-111), it was shown that the integration of the pO2 of the wound tissue increases collagen deposition and reduces infection. Published mechanistic data document that oxygen can generate a sustained increase in the pO2 of the wound tissue and of angiogenesis and, in chronic human wounds, can induce a progressive and sustained increase in VEGF expression.

The inflammatory state can also present in common oral pathological conditions, such as, for example, gingivitis and periodontitis.

Gingivitis is caused by the accumulation of bacterial plaque on the teeth, which induces a local inflammatory response in the gingival tissues. Plaque is a biofilm formed mainly by bacteria, which accumulates on the surface of the teeth following poor oral hygiene. The bacteria within the plaque produce toxins and other by-products that irritate the gums, causing redness, swelling and bleeding on probing (BOP). Gingivitis, being a condition limited to the gingival tissues, does not cause irreversible damage to the supporting tissues of the tooth, such as the bone and the periodontal ligament.

One of the main risk factors for the development of gingivitis is poor oral hygiene, but there are other predisposing factors, such as hormonal changes (for example during puberty or pregnancy), systemic diseases (such as diabetes), smoking and stress. It is important to underline that gingivitis is a reversible condition, in the sense that, by improving oral hygiene habits and removing plaque with professional scaling, the inflammation can be completely resolved.

Periodontitis is a severe form of gingival disease that affects the tissues that surround and support the teeth. This condition is caused by pathogens, especially bacteria, which accumulate on the surface of the teeth and in the gingival pockets, leading to inflammation and damage to the tissues. The main cause of periodontitis is the formation of bacterial plaque on the teeth.

The link between gingivitis and periodontitis is fundamental in understanding the pathogenesis of periodontal diseases. Gingivitis is the precursor to periodontitis: without gingival inflammation, there can be no progression to periodontitis. However, not all people with gingivitis necessarily develop periodontitis. The progression from gingivitis to periodontitis depends on a series of factors that go beyond the mere presence of bacterial plaque.

Among the factors that influence the progression of the disease are: the genetic susceptibility of the individual, the immune response, the systemic health status, smoking and other environmental factors. People with a weakened immune system or with chronic diseases such as diabetes have a higher risk of developing periodontitis. Furthermore, it has been shown that smoking increases the risk of progression from gingivitis to periodontitis, compromising the local immune response and reducing the supply of oxygen to the periodontal tissues.

Recently, the possibility of using topical oxygen therapy to counteract the growth of the bacterial biofilm of dental plaque has been introduced. Recent studies have shown that the use of topical oxygen-based therapy produces significant clinical results in the treatment of patients with gingivitis and periodontitis (Basudan AM, Abas I, [Translator's Note: "Sacido ialuronicoheen MY, Algacido ialuronicomdi HS" appears to be a transcription error in the original text]. Effectiveness of Topical Oxygen Therapy in Gingivitis and Periodontitis: Clinical Case Reports and Review of the Literature. J Clin Med. 2024 Mar 2;13(5):1451. doi: 10.3390/jcm13051451). A clear resolution of the signs of inflammation, a significant reduction in bleeding on probing (BOP) and a decrease in periodontal probing depth (PPD) have been observed, results similar to those reported for other adjunctive local treatments in the scientific literature. On the basis of the cases examined and the results reported, it can be concluded that topical oxygen-based therapy is effective in the management of gingivitis and periodontitis.

On the other hand, hyaluronic acid has been used in periodontal therapy in recent years. Hyaluronic acid is a fundamental component of the extracellular matrix in almost all body tissues and plays an active role throughout the wound healing process, participating in cell proliferation, migration and tissue remodelling (Chen W.Y., Abatangelo G. Functions of hyaluronan in wound repair. Wound Repair Regen. 1999;7:79-89. doi: 10.1046/j.1524-475X.1999.00079.x; Asparuhova M.B., Kiryak D., Eliezer M., Mihov D., Sculean A. Activity of two hyaluronan preparations on primary human oral fibroblasts. J. Periodontal Res. 2019;54:33-45. doi: 10.1111/jre.12602). Furthermore, it has been shown that hyaluronic acid has bacteriostatic, fungistatic, anti-inflammatory, anti-edematous, osteoinductive and pro-angiogenetic properties. In periodontal tissues, hyaluronic acid is synthesized by fibroblasts and keratinocytes in the gums, as well as by cells of the periodontal ligament, by cementoblasts and by osteoblasts (Dahiya P., Kamal R. Hyaluronic Acid: A boon in periodontal therapy. N. Am. J. Med. Sci. 2013;5:309-315. doi: 10.4103/1947-2714.112473). Furthermore, it has been shown how hyaluronic acid maintains the vitality of oral fibroblasts, improving their proliferative and migratory capacity.

Hyaluronic acid has been used in both non-surgical and surgical periodontal treatment, and it can be stated that: hyaluronic acid represents a promising material for regenerative and reconstructive periodontal surgery (Jentsch H., Pomowski R., Kundt G., Göcke R. Treatment of gingivitis with hyaluronan. J. Clin. Periodontol. 2003;30:159-164. doi: 10.1034/j.1600-051X.2003.300203.x; Eick S., Renatus A., Heinicke M., Pfister W., Stratul S.I., Jentsch H. Hyaluronic Acid as an adjunct after scaling and root planing: A prospective randomized clinical trial. J. Periodontol. 2013;84:941-949. doi: 10.1902/jop.2012.120269), in addition to promoting faster wound healing when used in oral soft tissue wounds (Romeo U., Libotte F., Palaia G., Galanakis A., Gaimari G., Tenore G., Del Vecchio A., Polimeni A. Oral soft tissue wound healing after laser surgery with or without a pool of amino acids and sodium hyaluronate: A randomized clinical study. Photomed. Laser Surg. 2014;32:10-16. doi: 10.1089/pho.2013.3509).

### Problem of the known art

For the treatment of inflammatory states and/or lesions of the oral cavity, local therapies can be used. For example, antibiotics and chlorhexidine are among the most commonly used agents; however, their prolonged use is associated with various side effects, including teeth pigmentation, cellular cytotoxicity and hypersensitivity reactions. Even anti-inflammatory drugs, as is known, are not immune from side effects.

In light of the state of the art and the need to find alternatives to traditional local therapies based on anti-inflammatory drugs and/or antibiotics, the need was felt to identify an association of at least two active substances capable of treating inflammatory states and/or continuous lesions of the oral cavity in a more efficient, safe, low-cost, non-invasive manner, and which can be easily administered.

### SUMMARY OF THE INVENTION

The Applicant has developed a topical oral association (or combination) comprising: molecular oxygen characterized by a degree of purity ≥ 90% by volume (v/v), and hyaluronic acid comprised in a formulation comprising suitable excipients and/or diluents, and which does not comprise molecular oxygen, for use in the treatment of inflammatory states of the oral cavity and/or continuous lesions of the oral cavity.

A further object of the invention is a kit comprising
- the association for the aforementioned use,
- means for delivering the molecular oxygen and means for applying the hyaluronic acid at a topical level, preferably at least one cannula and/or an airbrush.

### Advantages of the invention

The association of the invention is advantageous because:
- it effectively treats the inflammatory states of the oral cavity; and/or
- it effectively treats the continuous lesions of the oral cavity. In this sense, it is able to resolve the wound, reducing the inflammatory state and/or re-epithelializing the oral mucosa;
- it is non-invasive, nor are particular side effects observed;
- it is low-cost and easily applicable.

The molecular oxygen and the hyaluronic acid, comprised in the association of the invention support, promote and/or assist each other's action (reciprocal promotion of the biological activity of the two active ingredients).

### DETAILED DESCRIPTION OF THE INVENTION

Hereinafter, the invention and its preferred embodiments are described in greater detail. It should be noted that, although the structure has been organized into paragraphs and subparagraphs, the information contained in each paragraph or sub-paragraph is not isolated, and can be eventually combined with that contained in other paragraphs or, more generally, with other information contained in the text of the patent application.

### Association comprising molecular oxygen and hyaluronic acid

By "association" is meant the "combination" of two entities, even if these are not physically in the same formulation.

The association (or combination) of the invention comprises or consists of molecular oxygen and hyaluronic acid for use in the treatment of inflammatory states and/or continuous lesions of the oral cavity.

Preferably, the association (or combination) comprises or consists of molecular oxygen or in gaseous form and a formulation comprising, in turn:
- at least one active ingredient consisting of hyaluronic acid,
- suitable excipients and/or diluents,
and is for use in the treatment of inflammatory states and/or continuous lesions of the oral cavity.

### Molecular oxygen in gaseous form

The association of the invention comprises the active ingredient molecular oxygen (or gaseous). For the purposes of the invention, the molecular oxygen is not generated by a "generator" chemical compound, for example from peroxides.

The molecular oxygen (or gaseous oxygen) has a degree of purity comprised between ≥ 90% (v/v), preferably between 90% and 99%, preferably between 90% and 96% (v/v), preferably between 92% and 95% (v/v), preferably equal to 92% by weight, or to 93% by weight, or to 94% by weight, or to 95% by weight (v/v).

The molecular oxygen comprised in the association can be obtained using methodologies known to the person skilled in the art; preferably, the molecular oxygen is obtained thanks to an oxygen concentrator, which aspirates the external air and concentrates it in the oxygen fraction. The maximum delivery pressure by means of the oxygen concentrator is preferably comprised between 10 and 150 kPa, preferably between 30 and 150 kPa, preferably between 50 and 150 kPa, preferably it is 100 kPa.

The dosage of molecular oxygen to be administered is preferably comprised between 0.1 and 130 L, preferably between 0.5 and 130 L, preferably between 1 and 130 L, preferably between 5 and 130 L, preferably comprised between 5 and 120 L, preferably between 10 and 130 L, preferably comprised between 10 and 120 L, preferably between 20 and 120 L, preferably between 10 and 90 L, preferably between 10 and 70 L.

According to a preferred form of the invention, the molecular oxygen is delivered at a flow comprised preferably between 0.1 L/min and 7 L/min, preferably between 0.5 L/min and 7 L/min, preferably between 1 L/min and 6 L/min, preferably between 2 L/min and 6 L/min, preferably equal to 2 L/min or 6 L/min.

According to an alternative preferred form, the molecular oxygen is delivered at a flow comprised preferably between 0.05 L/min and 2 L/min, preferably between 0.1 L/min and 1 L/min, preferably between 0.1 L/min and 0.5 L/min.

Preferably, the molecular oxygen is delivered at a pressure comprised between 0.2 and 1.5 atm, preferably between 0.4 and 1 atm, preferably between 0.6 and 1 atm, preferably of 1 atm. Preferably, the parameters of a hyperbaric system (O₂ at 100% in a closed system) are not reached.

### Hyaluronic acid and formulation comprising hyaluronic acid

The association comprises a second active principle consisting of hyaluronic acid.

The hyaluronic acid is comprised in a formulation, the latter not comprising molecular oxygen or in gaseous form.

Preferably, the hyaluronic acid is the only active ingredient comprised in the formulation, the latter comprising suitable excipients and/or diluents.

Preferably, the hyaluronic acid comprised in the formulation is in a quantity comprised between 0.01% and 5% by weight, 0.05% and 5% by weight, preferably between 0.05% and 4% by weight, preferably between 0.07% and 4% by weight, preferably between 0.1% and 4% by weight, preferably between 0.5% and 4% by weight, preferably between 1% and 4% by weight, preferably between 1% and 3% by weight, preferably between 2% and 3% by weight, on the total weight of the formulation.

Preferably, the hyaluronic acid preferably has a molecular weight comprised between 100 kDa and 10 MDa, preferably between 100 kDa and 5 MDa, preferably between 100 kDa and 1 MDa, preferably between 100 kDa and 500 kDa, preferably between 100 kDa and 250 kDa, preferably between 180 kDa and 250 kDa, preferably between 180 kDa and 200 kDa.

It is known that hyaluronic acid with a molecular weight > 5 MDa is present in a semi-solid formulation, preferably a gel.

Preferably, the hyaluronic acid is in cross-linked form (cross-linked hyaluronic acid). Preferably, known cross-linking agents are: 1,4-butanediol diglycidyl ether (BDDE) and poly(ethylene glycol) diglycidyl ether (PEGDE). Preferably, the hyaluronic acid is comprised in a liquid or semi-solid formulation.

### 1st embodiment: hyaluronic acid comprised in the liquid formulation

Preferably, the hyaluronic acid is comprised in a liquid formulation.

The liquid formulation, preferably in the form of an aqueous solution, comprises or consists of:
- the active ingredient hyaluronic acid, and
- suitable excipients and/or diluents.

Preferably, the liquid formulation can be in the form of a nebulizable solution or spray.

Preferably, the liquid formulation is applied with a means suitable for nebulization, for example a cannula or an airbrush.

The liquid formulation is preferably for topical use, preferably it is applied at the level of the local inflamed and/or lesioned site of the oral cavity.

Preferably, suitable excipients and/or diluents for the preparation of aqueous solutions are known to the person skilled in the art. For example, excipients/diluents selected from the group consisting of: solvents (water), humectants, conditioning agents, chelating agents, antistatic agents, emulsifiers, surfactants, preservatives, buffer systems, pH modifiers, flavouring agents, deodorants, rheological agents, antioxidants can preferably be included.

Preferably, the excipients and/or diluents are selected from the group consisting of (INCI names): aqua, disodium EDTA, PVP (or Polyvinylpyrrolidone), [Translator's Note: "phenoxyetacido ialuroniconol" appears to be a transcription error in the original text, likely "phenoxyethanol"], benzoic acid, dehydroacetic acid, ethylhexylglycerin, sodium hydroxide, lactic acid, parfum, benzyl alcohol, methyl benzoate, Butylhydroxytoluene (BHT), PEG-40 hydrogenated castor oil, cross-linking agents (for example BDDE), and mixtures of the foregoing.

Preferably, the dosage of hyaluronic acid is comprised between 10 mg and 120 mg per dosage unit.

In the liquid formulation, the hyaluronic acid preferably has a molecular weight comprised between 40 kDa and 1400 kDa.

According to a preferred form, the liquid formulation comprises: hyaluronic acid with a molecular weight comprised between 40 kDa and 60 kDa, and hyaluronic acid with a molecular weight comprised between 1000 kDa and 1400 kDa.

The quantity of water comprised in the liquid formulation is comprised between 95% and 98% by weight, preferably between 96% and 98% by weight, on the total weight of the liquid formulation.

The liquid formulation is preferably in the form of a vial, preferably single-use; preferably, each hyaluronic acid vial has a volume comprised between 10 and 20 ml, preferably of 15 ml.

### 2nd embodiment: hyaluronic acid comprised in the semi-solid formulation

A semi-solid formulation is preferably in the form of a gel, emulgel, foam, mousse.

Preferably, the hyaluronic acid is comprised in a semi-solid formulation.

The semi-solid formulation, preferably in the form of a gel, comprises or consists of:
- the active ingredient hyaluronic acid, and
- suitable excipients and/or diluents.

The semi-solid formulation is preferably for topical use, preferably it is applied at the level of the local inflamed and/or lesioned site of the oral cavity.

This semi-solid formulation can contain excipients and/or diluents, selected from the group consisting of: chelating agents, surfactants, emulsifiers, humectants, solubilizers, rheological agents, perfumes, preservatives, buffer systems, pH modifiers, cross-linking agents (for example, BDDE), and mixtures of the foregoing.

The Applicant believes that the hyaluronic acid comprised in the formulation exerts its combined effect with that of the oxygen in gaseous form at the oral topical level *independently* of the embodiment of the formulation comprising the hyaluronic acid.

### Use of the association of the invention

The association comprising molecular oxygen and hyaluronic acid is for use in the treatment of inflammatory states of the oral cavity and/or continuous lesions of the oral cavity.

Preferably, the inflammatory states of the oral cavity manifest concurrently with or are caused by conditions selected from the group consisting of: stress or thermal or mechanical traumas or, further, induced by irritating substances; surgical interventions; dental plaque accumulation; reduced immune defences; pathogens; inflammatory diseases of the oral cavity; lesions of the oral cavity; and combinations of the foregoing.

More preferably, the inflammatory states of the oral cavity manifest concurrently with or are caused by conditions selected from the group consisting of: surgical interventions; dental plaque accumulation; pathogens; inflammatory diseases of the oral cavity; lesions of the oral cavity; and combinations of the foregoing.

Preferably, the association comprising molecular oxygen and hyaluronic acid is for use in the treatment of inflammatory diseases of the oral cavity.

Preferably, the inflammatory diseases of the oral cavity are selected from the group consisting of: gingivitis; periodontitis; periodontal diseases; and combinations of the foregoing.

More preferably, the periodontal diseases are selected from the group consisting of: peri-implant mucositis; peri-implantitis; and combinations of the foregoing.

It is known that periodontal diseases, such as peri-implantitis, have common causes with periodontitis, but arise in the presence of dental implants; furthermore, the biological and pathogenic mechanisms between periodontal diseases and periodontitis are the same.

By continuous lesions (or solutions of continuity) of the oral cavity are meant more or less deep interruptions of the anatomical continuity of the oral mucosa and/or the oral epithelium. In this sense, the association is able to promote the regeneration or re-epithelialization of the oral epithelium and/or the oral mucosa.

Preferably, by continuous lesions of the oral cavity, preferably of the oral mucosa and/or the oral epithelium, are meant lesions/wounds that manifest concurrently with or are caused by conditions selected from the group consisting of: thermal or mechanical traumas or stresses; surgical interventions; pathogens; inflammatory diseases; and combinations of the foregoing.

Preferably, the continuous lesions of the oral cavity are post-surgical lesions.

Preferably, the association is for oral topical use, preferably it is applied locally at the site of inflammation, preferably it is applied locally at the level of the oral mucosa.

Preferably, the association is for use as an *adjuvant* in the treatment of inflammatory states of the oral cavity and/or in the regeneration of the oral epithelium and/or the oral mucosa.

For the aforesaid purposes, by adjuvant is meant the association of the invention which, in combination with at least one medical therapy known for the aforesaid purposes (for example, an anti-inflammatory therapy), completes and/or increases its therapeutic action efficacy.

More preferably, the association is used in combination with at least one dental cleaning practice, for example mechanical dental cleaning, known in the dental field.

### Kit comprising the association of the invention

A further object of the invention is a kit, preferably a kit for use in the treatment of inflammatory states and/or continuous lesions of the oral cavity, comprising:
- the association of the invention comprising molecular oxygen and hyaluronic acid, or the association comprising oxygen in gaseous form and the formulation comprising the active ingredient hyaluronic acid and suitable excipients and/or diluents,
- preferably, at least one generator of molecular oxygen (or in gaseous form),
- preferably, the formulation comprising hyaluronic acid, preferably in semi-solid or liquid form,
- means for applying and/or delivering the molecular oxygen at the oral topical level, preferably at least one cannula or an airbrush,
- optionally, connection means, preferably connection means between the at least one generator of oxygen in gaseous form and the means for delivering the molecular oxygen at the oral topical level, for example a cannula or an airbrush,
- optionally, means for regulating the flow of molecular oxygen, preferably a flowmeter,
- optionally, instructions for the kit and/or its use.

It should be noted that the connection means can preferably be tubes made of a material suitable for containing a flow of molecular oxygen or in gaseous form, preferably plastic tubes.

### Therapeutic protocol

Preferably, the method according to the invention comprises the following steps:
- delivering the molecular oxygen in gaseous form at the oral topical level or step (a);
- *subsequently* to the aforesaid delivery step (a), applying the hyaluronic acid at the oral topical level or step (b), preferably applying topically a formulation comprising hyaluronic acid, the formulation being preferably in liquid form (for example, an aqueous solution) or semi-solid form (for example, a gel),
   *or, alternatively,*
- delivering the molecular oxygen in gaseous form at the oral topical level, and *concurrently also* applying the hyaluronic acid, preferably applying topically a formulation comprising hyaluronic acid, the formulation being preferably in liquid form (for example, an aqueous solution) or semi-solid form (for example, a gel).

Preferably, the method or protocol is performed daily, preferably once a day.

Preferably, the method or protocol is performed for a time comprised between 1 and 30 days, preferably between 1 and 15 days.

### 1st preferred embodiment

Preferably, the method according to the invention comprises the following steps:
- delivering the molecular oxygen in gaseous form at the oral topical level or step (a);
- *subsequently* to the aforesaid delivery step (a), applying the hyaluronic acid at the oral topical level or step (b), preferably applying topically a formulation comprising hyaluronic acid, the formulation being preferably in liquid form (for example, an aqueous solution) or semi-solid form (for example, a gel).

According to this first preferred form, the step (a) of delivering the molecular oxygen in gaseous form at the oral topical level has a duration comprised between 5 seconds and 5 minutes, preferably between 5 seconds and 3 minutes, preferably between 5 seconds and 1 minute, preferably between 5 and 50 seconds, preferably between 10 and 40 seconds, preferably between 20 and 30 seconds, preferably of 30 seconds.

Preferably, the aforesaid step (a) provides for the delivery of the active ingredient oxygen in gaseous form at the topical level, at the local site of inflammation and/or lesion, preferably through the use of at least one suitable delivery means, such as for example a cannula, an airbrush and/or a syringe.

Preferably, the step (b) of applying the hyaluronic acid at the oral topical level is subsequent to the aforesaid step (a); the step (b) of applying the hyaluronic acid at the oral topical level preferably provides for the topical application of a formulation comprising hyaluronic acid, the formulation being preferably in liquid form (for example, an aqueous solution) or semi-solid form (for example, a gel).

Preferably, the step (b) of applying the hyaluronic acid at the oral topical level occurs at the topical level, preferably at the inflamed and/or lesioned site where the molecular oxygen of step (a) was previously delivered, until complete absorption.

Preferably, the step (b) of applying the hyaluronic acid at the oral topical level does not require rinsing.

Preferably, the step (b) of applying the hyaluronic acid at the oral topical level occurs through the use of at least one suitable means, such as for example a syringe.

### 2nd preferred embodiment

In accordance with a second preferred embodiment, alternative to the first previously described, the method provides for
- delivering the molecular oxygen in gaseous form at the oral topical level, and
- *concurrently* applying the hyaluronic acid, preferably applying topically a formulation comprising hyaluronic acid, the formulation being preferably in liquid form (for example, an aqueous solution) or semi-solid form (for example, a gel).

Preferably, the association of molecular oxygen and hyaluronic acid is applied at the oral topical level, preferably at the level of the site of inflammation, preferably through the use of at least one suitable means, such as for example an airbrush.

Preferably, the association of molecular oxygen and hyaluronic acid is applied at the oral topical level for a duration comprised between 0.5 and 10 minutes, preferably between 1 and 10 minutes, preferably between 2 and 10 minutes, preferably between 2 and 7 minutes, preferably between 2 and 5 minutes, preferably of 4 minutes.

### EXAMPLES

Hereinafter, the Applicant sets forth examples for illustrative but not limitative purposes.

It should be noted that, in the following examples, the expression "association" refers to the association of molecular oxygen and hyaluronic acid object of the invention.

For "oxygen considered individually" and for "hyaluronic acid considered individually" are meant respectively the oxygen and the hyaluronic acid not included in the scope of the association of the invention.

### Example 1 - Treatment of residual periodontal pockets with subgingival irrigation of oxygen and hyaluronic acid

The objective of the study is to evaluate the clinical efficacy of the adjunctive local therapy based on the association of molecular oxygen and hyaluronic acid in the treatment of residual pockets in subjects affected by periodontitis.

By adjunctive local therapy is meant that the association is used in combination with the mechanical cleaning practices known in the dental field.

This study was designed as a prospective, split-mouth, single-blind, randomized and controlled clinical study to compare the association between local oxygenation therapy and hyaluronic acid; local oxygenation; local hyaluronic acid in the treatment of residual pockets in subjects with periodontitis.

The adjunctive local oxygenation therapy is delivered through a technology that uses a device for the administration of oxygen, supplied by a special airbrush that modulates a flow of oxygen > 90%.

The oxygen is delivered into the periodontal pocket through a single-use blunt cannula (1 mm in diameter) mounted on a plastic syringe. Once the device is activated, the cannula will be inserted to the bottom of the pocket and then moved 1-2 mm in the coronal direction.

The site will be oxygenated for 30 seconds, with a flow of 2 L/min and a pressure of 1 atm.

The hyaluronic acid is applied delicately in the form of a gel product containing hyaluronic acid, in which the latter is in a quantity equal to 3% by weight on the total weight of the gel product, using a sterile syringe with a blunt tip until it overflows from the periodontal pocket. In particular, the quantity of hyaluronic acid-based gel that is applied is a function of the size of the pocket, for example it can range from 10 µl up to 1 ml.

It should be noted that, within the scope of the association, the methods and parameters of delivery/application of the oxygen and the hyaluronic acid are the same as those for the oxygen, considered individually, and for the hyaluronic acid, considered individually.

Within the scope of the association, once the oxygenation has been carried out, the gel containing hyaluronic acid is applied delicately using a sterile syringe with a blunt tip until it overflows from the periodontal pocket.

*Clinical measurements*: an examiner other than the operator, unaware of the therapies administered, will perform all the clinical measurements. To calibrate the examiner, five subjects not part of the study with intra-bony defects were selected. The examiner will measure the periodontal probing depth (PPD), the primary variable, in all patients twice within 24 hours. At the end of the training session, the intra-class correlation coefficient for the PPD must be greater than 0.75; otherwise, the examiner will be retrained and re-evaluated. At baseline, the following clinical parameters will be recorded at 6 sites per tooth with a manual periodontal probe graduated at 1 mm (PCP-UNC 15, Hu-Friedy Manufacturing Co.) and the target sites will be defined.

The measurements will be repeated at 1 month and at 3, 6 and 12 months after the retreatment of the residual pockets:
- Plaque index (PI), using the Ainamo & Bay index;
- Probing depth (PD);
- Bleeding on probing (BoP), using the Ainamo & Bay index;
- Recession depth (rec);
- Clinical attachment level (CAL), calculated as PD+Rec;
- Tooth mobility, according to the Lindhe classification;
- Total plaque index/Bleeding index.

At each follow-up, the patient will be asked to indicate the level of pain by marking on a 100 mm VAS scale. The evaluation will be recorded as the distance from the left side of the scale (0 mm) to the mark made by the patient. The patient will be invited to report the pain both in the treated site and in the untreated one.

*Objective and results*: the objective of this randomized clinical study is to investigate the clinical effect of the adjunctive local therapy with oxygen and hyaluronic acid and their combination in the treatment of subjects with periodontitis in terms of the following outcome variables:
- *primary variables*: change in periodontal probing depth (PD cacido ialuroniconge);
- *secondary variables*: reduction of bleeding on probing (BoP red); gain in clinical attachment level (CAL gain); patient-reported outcome measures (PROMs) (questionnaire); reduction of the load of periodontal pathogens.

Example 2 - Evaluation of the effects of the treatment with oxygen and hyaluronic acid in wounds of connective-epithelial tissue, in particular of tissue taken from the palate.

The study concerns the evaluation of the effects of the treatment with high-concentration oxygen and hyaluronic acid of connective-epithelial tissue taken from the palate in patients selected for a surgical treatment of keratinized tissue augmentation with a free gingival graft.

Within the scope of this study, the evaluation is also carried out considering the comparison between the untreated tissue ("control") and the tissue treated with the association.

A portion of the graft, not useful for the graft, will be divided into two equal parts. The randomized allocation of the treatment with extra-oral oxygen (test site) and without oxygen (control) will be established by tossing a coin. Both portions of the graft will be subjected to histological analysis.

After performing local anesthesia by infiltration (2% lidocaine with epinephrine 1:100,000) distant from the recipient site of the graft, to avoid edema at the surgical site, a partial thickness flap will be performed. The design of the flap will be a horizontal incision, in the mesio-distal direction, of about 14 mm at the level of the mucogingival junction, followed by two corono-apical releasing incisions, at its ends, of about 7 mm. The elevated flap will be removed preserving the periosteum. A gauze soaked in isotonic solution will be placed to cover the recipient site until the positioning of the graft.

The palatal graft of standard dimensions (14 x 7 mm) will then be taken in the area between the first premolar and the first molar, at 3 mm from the gingival margin of the corresponding teeth. The donor area will be cleansed with sterile saline solution and sutured (5-0 monofilament nylon) to stabilize the clot. The epithelial-connective graft will be prepared by removing any irregularities and the adipose tissue and, if necessary, thinned to obtain a uniform thickness of about 1.5 mm.

The graft will be divided into three parts, according to its length, and the central portion, of about 10 mm, will be adapted and firmly sutured (6-0 monofilament nylon) to the recipient site with suspended periosteal sutures and single sutures at the keratinized gingiva. The lateral portions of the graft, of about 2 mm each, will be randomized, with the toss of a coin, to receive the treatment with extra-oral oxygen (test) or no treatment (control). Both will be subjected to histological analysis.

The topical oxygenation is carried out by delivering pure O₂ at 93±3%, for a flow rate of 6 liters/minute at atmospheric pressure (0.101325 Mpa). The flow of O₂ generated has a pressure of 0.04-0.07 Mpa without reaching the parameters of a hyperbaric system (O₂ at 100% in a closed system). Currently the device is used in the treatment of respiratory, cardiovascular, cerebrovascular pathologies and in non-invasive medical aesthetic treatments of the skin.

The hyaluronic acid is applied by means of a product in the form of a gel, in which the hyaluronic acid is in a quantity equal to 3% of the total weight of the gel.

The oxygen considered individually and the hyaluronic acid considered individually are applied following the same methods and the same parameters of the oxygen and the hyaluronic acid used within the scope of the association.

Immediately after the surgery, the test sample will be subjected to topical oxygenation with hyaluronic acid, i.e. subjected to the association of the invention. The association will be administered for 4 minutes by means of a special airbrush positioned at a distance of 1-2 mm from the tissue (nebulization of molecular oxygen and hyaluronic acid).

*Histological analysis*: The tissue samples fixed in 10% buffered formalin were included in paraffin; from the block thus obtained, consecutive histological sections of 3 µm thickness were prepared, stained both with the routine histochemical staining of hematoxylin-eosin for the evaluation of histomorphological changes, and with the histochemical staining of Picrosirius (Sirius Red) for the study of collagen tissue, in which the latter staining is also analyzed with polarized light (87 A B Staining with Sirius Red (A-dark field; B-bright field).

From the same paraffin blocks, further histological sections were prepared for the characterization of the inflammatory infiltrate and of neoangiogenesis. To this end, immunohistochemical stainings were performed with ready-to-use Leica antibodies:
- anti CD3 antibody (specific marker of T lymphocytes);
- anti CD20 antibody (specific marker of B lymphocytes);
- anti CD68 antibody (specific marker of macrophages);
- anti CD34 antibody (marker of endothelial cells).

The reaction was amplified and visualized using the Leica Bond III immunostainer with Bond Polymer Refine Detection.

Two pathologists, independently and blind to the correspondence of the analyzed samples, will evaluate in a semi-quantitative manner the intensity of the inflammatory infiltrate (*score*: absent, mild, moderate and intense).

The vascular density will instead be evaluated, after digital acquisition of the immunostained slides with an Aperio scanner (Leica Biosystems), with ImageJ software by quantifying the positive signal in 5 fields at 20X magnification:
- the number of vessels present in the connective tissue of the corium (expressed as mean ± SD);
- the caliber of the vessels (area occupied by the vessel in µm, expressed as mean ± DS);
- the microvessel density (expressed as number of vessels over the total analyzed area equal to 6.31 mm²).

## Claims

1. Oral topical association comprising
- molecular oxygen **characterized by** a degree of purity ≥ 90% by volume (v/v), and
- hyaluronic acid comprised in a formulation containing suitable excipients and/or diluents, and which does not comprise molecular oxygen,
for use in the treatment of inflammatory states of the oral cavity and/or continuous lesions of the oral cavity.

2. Association for use according to claim 1, wherein the inflammatory states of the oral cavity manifest concurrently with or are caused by conditions selected from the group consisting of: thermal or mechanical stresses or traumas or, further, induced by irritating substances; surgical interventions; dental plaque accumulation; reduced immune defences; pathogens; inflammatory diseases of the oral cavity; lesions of the oral cavity; and combinations of the foregoing.

3. Association for use according to claim 1 or 2, wherein the inflammatory diseases of the oral cavity are selected from the group consisting of: gingivitis; periodontitis; periodontal disease; and combinations of the foregoing.

4. Association for use according to any one of claims 1 to 3, wherein the molecular oxygen dosage is comprised between 0.1 and 130 litres.

5. Association for use according to any one of claims 1 to 4, wherein the formulation, comprising the hyaluronic acid, is in a liquid or semi-solid formulation.

6. Association for use according to any one of claims 1 to 5, wherein the hyaluronic acid is comprised in the formulation in an amount comprised between 0.01% and 5% by weight on the total weight of the formulation.

7. Association for use according to claim 5 or 6, wherein the hyaluronic acid is the only active ingredient comprised in the formulation.

8. Association for use according to any of claims 1 to 7, wherein the continuous lesions of the oral cavity are lesions/wounds that occur in conjunction with or are caused by conditions selected from the group consisting of: thermal or mechanical trauma or stress; surgical interventions; pathogens; inflammatory diseases; and combinations of the foregoing.

9. Kit comprising
• the association for use according to any one of claims 1 to 8,
• means for delivering the molecular oxygen and means for applying the hyaluronic acid at oral topical level, preferably at least one cannula and/or spray gun.
